# EUROPEAN PATENT SPECIFICATION

(11) **EP 4 395 731 B1**
(45) Date of publication and mention of the grant of the patent: **02.04.2025**
(21) Application number: 22764791.4
(22) Date of filing: 16.08.2022
(51) Int. Cl.: A61K 8/06, A61K 8/41, A61K 8/46, A61K 8/49, A61Q 5/00, A61Q 5/02, A61Q 5/12

(54) **HAIR CARE COMPOSITION**
HAARPFLEGEZUSAMMENSETZUNG
COMPOSITION DE SOINS CAPILLAIRES

(30) Priority: 31.08.2021 WO PCT/CN2021/115834; 13.10.2021 EP 21202433
(43) Date of publication of application: 10.07.2024
(73) Proprietor: Unilever IP Holdings B.V., 3013 AL Rotterdam (NL); Unilever Global IP Limited, Wirral, Merseyside CH62 4ZD (GB)
(72) Inventor: AO, Mingqi, 6708 WH Wageningen (NL); LE, Yingyi, 6708 WH Wageningen (NL); LI, Zhengrong, 6708 WH Wageningen (NL)
(74) Representative: Mathai, Neenu Grace
(86) International application number: PCT/EP2022/072798
(87) International publication number: WO 2023/030871

(56) References cited:
- EP-A1- 3 695 825
- WO-A1-2021/144267
- WO-A1-2021/144326
- WO-A1-2021/175499

## Description

### Field of the Invention

This invention relates to hair care compositions, more particularly to hair care compositions comprising anti-dandruff agents. The present invention also relates to a method of improving deposition of anti-dandruff agents, especially during topical application to the scalp

### Background of the Invention

Hair care compositions generally provide cleansing or conditioning benefits or a combination of the two. Such compositions typically comprise one or more cleansing surfactants which generally aid in cleaning the hair and the scalp free of undesirable soil, particles and fatty matter.

Dandruff control is an important aspect of hair cleansing and care. Anti-dandruff benefit has been provided through hair care compositions including shampoos and hair conditioners. Dandruff is an issue that affects many people globally. The condition is manifested by the shedding of clumps of dead skin cells from the scalp. These are white in colour and provide an aesthetically displeasing appearance. A factor that contributes to dandruff is certain members of the Malassezia yeasts. To combat these, anti-dandruff products have included certain anti-dandruff agents which have anti-fungal activity, for example, piroctone compound such as piroctone olamine. These anti-dandruff agents remove (or at least reduce the level of) the Malassezia from the scalp and provide moderately effective treatment of the dandruff condition. Such a product performs as a hair cleansing shampoo or as a hair conditioner while mitigating the causes of dandruff.

A common problem with piroctone compound is that deposition of the active onto the desired surface during wash process is minimum. The desired surface is typically scalp and/or hair. For example, piroctone compound such as piroctone olamine is usually soluble in surfactants of the cleansing phase comprised in a hair care composition. During the excessive rinsing process, the majority of piroctone is likely to be washed away together with the surfactants. Poor deposition is correlated with low antidandruff activity, thus little mitigation of the ill-effects of dandruff. To date, there are attempts to offset this drawback by increasing the level of piroctone olamine in hair care composition. Such approach causes a variety of issues such as increased costs, potential instability of the formulation and potential adverse effect to hair sensory. Hence it is not an approach favoured by the industry.

Consequently, there remains a need to improve the deposition of piroctone compounds, onto the surface of scalp and/or hair during washing process. There also remains a further need to improve the deposition onto said surface without adverse side effects to sensory, formulation rheology and conditioning performance.

### Tests and Definitions

### Hair Care Composition

"Hair care composition", as used herein, is meant to include a composition for topical application to hair and/or scalp of mammals, especially humans. Such a composition may be generally classified as leave-on or rinse off, and includes any product applied to a human body for also improving appearance, cleansing, odor control or general aesthetics. The composition of the present invention can be in the form of a liquid, lotion, cream, foam, scrub, gel, or bar. Non-limiting examples of such compositions include leave-on hair lotions, creams, and rinse-off shampoos, conditioners, shower gels, or toilet bar. The composition of the present invention is preferably a rinse-off composition, especially preferred being a shampoo or a conditioner and most preferably a shampoo.

### Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final hair care composition, unless otherwise specified. It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Summary of the Invention

In a first aspect, the present invention is directed to a hair care composition comprising:
i) a cleansing surfactant comprising an amphoteric surfactant and an anionic surfactant;
ii) a hydroxamic acid or salt thereof having a carbon chain of C4 to C12 in an amount of from 0.01 to 10% by weight of the total composition;
iii) an oil phase comprising at least one liquid hydrocarbon oil in an amount of from 0.01 to 10% by weight of the total composition; and
iv) a piroctone compound;
wherein the weight ratio of the amount of the combination of said hydroxamic acid or salt and said liquid hydrocarbon oil to the amount of said piroctone compound is from 1:1 to 8:1; wherein said hydroxamic acid is caprylhydroxamic acid.

In a second aspect, the present invention is directed to a non-therapeutic method of treating the scalp or hair comprising the step of applying a composition of the first aspect onto the scalp or hair.

In a third aspect, the present invention is directed to a method of depositing piroctone compound onto scalp comprising the step of applying the hair care composition of the first aspect of this invention onto scalp surfaces of an individual.

In a fourth aspect, the present invention is directed to a composition of the first aspect for use in preventing or alleviating the symptoms of dandruff on the scalp or hair.

All other aspects of the present invention will more readily become apparent upon considering the detailed description and examples which follow.

### Detailed Description of the Invention

### Piroctone compound

The piroctone compound for use in the present invention include piroctone acid, primary, secondary and tertiary olamine salts of piroctone acid (such as the diethanolamine and triethanolamine salts), and mixtures thereof, preferably piroctone acid, primary olamine salt of piroctone acid (i.e. piroctone olamine, also known as Octopirox^{®}) and mixtures thereof. Most preferably the piroctone compound is piroctone olamine.

Piroctone olamine is an olamine salt of the hydroxamic acid derivative piroctone. It is commonly known as piroctone ethanolamine with the trade name Octopirox^{®}.

The piroctone olamine according to the present invention is a 1:1 compound of 1-hydroxy-4-methyl-6-(2,4,4-trimethylpentyl)-2(*1H*)-pyridinone with 2-aminoethanol and is also designated 1-hydroxy-4-methyl-6-(2,4,4-trimethylpentyl)-2(*1H*) pyridinone monoethanolamine salt. The CAS number is 68890-66-4 and the compound has the general formula (I) as below:

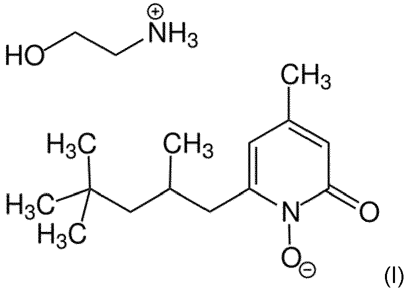

The piroctone compound, particularly the piroctone olamine is preferably present from 0.01 to 10% by weight of the total composition, more preferably from 0.1 to 5 wt%, most preferably from 0.2 to 3 wt% by weight of the total composition.

### Liquid hydrocarbon oil

The composition in accordance with the invention comprise an oil phase comprising at least one liquid hydrocarbon oil. Preferably the liquid hydrocarbon oil is a non-volatile hydrocarbon oil. Oils can be selected from the group consisting of triglycerides and fatty esters, Preferably the non-volatile oil is selected from the group consisting of triheptanoin, tricaprylin, tricaprin, triundecanoin, trilinolein, triolein, almond oil, coconut oil, olive oil, palm kernel oil, peanut oil and sunflower oil. It is more preferred the oil is coconut oil or sunflower oil.

The composition comprises non-volatile hydrocarbon oil in an amount of from 0.01 to 10%, preferably from 0.1 to 5%, more preferably from 0.1 to 2%, by weight of the total composition.

### Hydroxamic acid or salt thereof having a carbon chain of C4-C12

A hydroxamic acid is a class of organic compounds bearing the functional group RC(O)N(OH)R', with R and R' as organic residues and CO as a carbonyl group.

The composition in accordance with the invention comprises a hydroxamic acid represented by the formula R-CH2-CO-NHOH (where R is an alkyl group or alkoxyphenyl group) or a salt thereof having a carbon chain of C4-C12, such as butyrohydroxamic acid, succinyl hydroxamic acid, benzohydroxamic acid, nicotinyl hydroxamic acid, caprylhydroxamic acid, or laurohydroxamic acid. The hydroxamic acid in accordance with the invention is caprylhydroxamic acid.

The composition of the present invention comprises a hydroxamic acid or a salt thereof having a carbon chain of C4-C12 in an amount of from 0.01 to 10%, preferably from 0.1 to 5%, more preferably from 0.1 to 2%, by weight of the total composition.

Without binding to a theory, it is believed that the combination of the liquid hydrocarbon oil and hydroxamic acid or salt thereof can decrease the solubilization of piroctone compound in surfactant micelles which in turn leads to an increase of the amount of piroctone compound available in solution, and then contributes to the higher deposition upon application.

In accordance with the present invention, the weight ratio of the amount of the combination of hydrocarbon oil and hydroxamic acid or salt thereof to the amount of the piroctone compound is from 1:1 to 8:1, preferably from 1:1 to 6:1, more preferably from 1:1 to 4.5:1, furthermore preferably from 1:1 to 3:1, most preferably from 1.5:1 to 2:1.

It is preferred that the weight ratio of the amount of hydrocarbon oil to the amount of the piroctone compound is from 1:2 to 3:1.

It is preferred that the weight ratio of the amount of hydroxamic acid or salt thereof to the amount of the piroctone compound is from 1:2 to 3:1.

### Cleansing surfactant

The composition in accordance with this invention comprises a cleansing surfactant comprising an anionic surfactant and an amphoteric surfactant.

Non-limiting examples of suitable anionic surfactants are alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule. Typical anionic surfactants for use in compositions of the invention include, but not limited to, sodium oleyl succinate, ammonium lauryl sulphosuccinate, sodium lauryl sulphate, sodium lauryl ether sulphate, sodium lauryl ether sulphosuccinate, ammonium lauryl sulphate, sodium dodecylbenzene sulphonate, triethanolamine dodecylbenzene sulphonate, sodium cocoyl isethionate, sodium lauryl isethionate, lauryl ether carboxylic acid, sodium N-lauryl sarcosinate or mixtures thereof.

Preferred anionic surfactants are the alkyl sulphates and alkyl ether sulphates. Preferred alkyl sulphates are C8-18 alky sulphates, more preferably C12-18 alkyl sulphates, preferably in the form of a salt with a solubilising cation such as sodium, potassium, ammonium or substituted ammonium. Examples are sodium lauryl sulphate (SLS) or sodium dodecyl sulphate (SDS). It is particularly preferred that the anionic surfactant is alkyl ether sulphate. Preferred alkyl ether sulphates are those having the formula: RO(CH₂CH₂O)ₙSO₃M; wherein R is an alkyl or alkenyl having from 8 to 18 (preferably 12 to 18) carbon atoms; n is a number having an average value of greater than at least 0.5, preferably between 1 and 3, more preferably between 1 and 2; and M is a solubilising cation such as sodium, potassium, ammonium or substituted ammonium. An example is sodium lauryl ether sulphate (SLES). Preferred alkyl ether sulphate is sodium lauryl ether sulphate having an average degree of ethoxylation of from 0.5 to 3, preferably from 1 to 3, more preferably from 1 to 2.

It is preferred that the anionic surfactant is an ethoxylated alkyl sulphate a degree of ethoxylation of less than 3, preferably an ethoxylated alkyl sulphate a degree of ethoxylation of less than 2, more preferably sodium laureth sulphate (1EO).

The anionic surfactants are typically present in hair care composition of the present invention at a level of from 0.5 to 20%, more preferably from 2 to 16% and most preferably from 3 to 16%, by weight of the hair care composition.

Suitable amphoteric surfactant examples include alkyl amine oxides, alkyl betaines, alkyl amidopropyl betaines, alkyl sulphobetaines (sultaines), alkyl amphoacetates, alkyl amphopropionates, alkyl amidopropyl hydroxysultaines, wherein the alkyl group has from 8 to 19 carbon atoms.

Preferably, the amphoteric surfactant is a betaine surfactant. It is preferred that the betaine surfactant is one of alkyl amidopropyl betaines. Cocamidopropyl betaine (CAPB) is particularly preferred.

In a preferred embodiment, the composition comprises from 0.1 to 10 wt.%, preferably from 0.5 to 8 wt.%, more preferably from 1 to 5 wt.% of an amphoteric surfactant by weight of the total composition.

The ratio of ethoxylated anionic surfactant to amphoteric surfactant in which the ratio of anionic surfactant to amphoteric surfactant is less than 10:1, preferably from 2:1 to 10:1, more preferably from 3:1 to 9:1.

The total amount of cleansing surfactant in a shampoo composition for use in the invention is generally from 3 to 35 wt%, preferably from 5 to 25 wt%, most preferably from 7 to 16 wt% of the total composition.

### Hair care composition

The hair care composition in accordance with the present invention comprises an aqueous cosmetically acceptable carrier.

In a preferred embodiment, the hair care composition is a shampoo, particularly a rinse off shampoo. Shampoo compositions for use in the invention are generally aqueous, i.e. they have water or an aqueous solution or a lyotropic liquid crystalline phase as their major component. Suitably, the shampoo composition will comprise from 50 to 98%, preferably from 60 to 92% water by weight based on the total weight of the composition. Such compositions are referred to as having an aqueous base.

Preferably, the composition in accordance with the invention comprises a cationic polymer.

The composition may also comprise a cationic modified guar polymer having a molecular weight of from 0.3 to 2.5 million Dalton and a cationic degree of substitution of from 0.05 to 0.4.

It is preferred that the cationic modified guar polymer has a molecular weight of from 1.0 to 2.3 million Dalton, more preferably from 1.0 to 1.5 million Dalton.

It is preferred that the cationic guar polymer has a cationic degree of substitution of from 0.1 to 0.3, more preferably from 0.16 to 0.2.

It is most preferred that cationic modified guar polymer has a molecular weight of from 1.0 million to 1.5 million Dalton and a cationic degree of substitution (DS) of from 0.16 to 0.20. This DS value corresponds to a charge density of from 0.85 to 1.05.

Cationic guar polymer is preferably guar hydroxypropyltrimonium chloride. Guar polymer predominantly contains galactomannan polymer chains. This polymer is available at various molecular weights and degree of cationic substitutions depending on how much the guar has been hydrolysed and cationised.

Generally for cationic polysaccharide polymers, the hydroxyl groups of the non-modified monomeric sugar ring units are the sites for cationic substitution. Degree of substitution (DS) is typically in the range from 0 to 3 due to the fact that the monomeric sugar unit of most polysaccharide has in average three hydroxyl groups available for substitution. In addition to the DS, the cationic charge on polymers can also be quantified as cationic charge density. DS has previously been determined by different methods. For example, the cationic charge density of the polymer has in some cases been calculated based on a percent nitrogen content determined via the Kjeldahl method as described in US Pharmacopoeia under chemical tests for nitrogen determination and is expressed in milliequivalents (meq) per gram. The cationic charge density of the polymer in the present invention is, however, calculated from the degree of substitution, which is measured by 1H NMR in a solvent of deuterium oxide (D20) and deuterium chloride (DCI) mixture.

In many cases the DS obtained from 1H NMR measurement may not be suitable to be compared with that obtained from Kjeldahl method, due to the fact that the two methods are influenced by different factors.

In the wide spectrum of molecular weights of guars available, the cationic guar for use in the present invention has a molecule weight which is considered a 'medium' molecular weight. In the wide range of charge densities, the above range for use in the present invention is considered a 'medium' range. The cationic modified guar deposition polymer is preferably present in an amount of from 0.001 to 2%, more preferably from 0.01 to 1%, in 0.04 to 0.5%, more preferably 0.08 to 0.25% by weight of the composition.

The compositions of the present invention may comprise a copolymer acrylamidopropyltrimonium chloride. Preferably, the copolymer also comprises acrylamide in addition to the acrylamidopropyltrimonium chloride. Particularly preferred copolymer is a copolymer of acrylamidopropyltrimonium chloride and acrylamide.

The copolymer according to the present invention preferably has a charge density of from 1.0 to 3.0 meq per gram (meq/g), more preferably from 1.1 to 2.5 meq/g, more preferably still from 1.2 to 2.5 meq/g and most preferably from 1.3 to 2.5 meq/g. The copolymer preferably has a molecular weight of from 100,000 to 3,000,000 gram per mole (g/mol), more preferably from 500,000 to 2,800,000 g/mol, more preferably still from 800,000 to 2,500,000 g/mol, most preferably from 1,000,000 to 2,500,000 g/mol. An example of a suitable copolymer is commercially available from Ashland under the trade name N-Hance SP-100^{®}. N-Hance SP-100° has a charge density of from 1.8 to 2.2 meq/g and a molecular weight of from 1,800,000 to 2,200,000 g/mol.

The hair care composition of the present invention may typically comprise the copolymer in an amount of from 0.001 to 2%, more preferably from 0.01 to 1%, even more preferably from 0.01 to 0.8% and most preferably from 0.05 to 0.5%, based on total weight of the hair care composition and including all ranges subsumed therein.

In addition to the copolymer, it is preferable that the hair care composition also comprises other cationic polymers. Suitable cationic polymers may be homopolymers or be formed from two or more types of monomers. The molecular weight of the polymer will generally be between 5,000 and 10,000,000 g/mol, typically at least 10,000 g/mol and preferably from 100,000 to 2,000,000 g/mol. The polymers will have cationic nitrogen containing groups such as quaternary ammonium or protonated amino groups, or a mixture thereof. The cationic nitrogen containing group will generally be present as a substituent on a fraction of the total monomer units of the cationic polymer. Thus when the polymer is not a homopolymer it can contain spacer non-cationic monomer units. The ratio of the cationic to non-cationic monomer units is selected to give a polymer having a cationic charge density in the required range.

Suitable cationic polymers include, for example, copolymers of vinyl monomers having cationic amine or quaternary ammonium functionalities with water soluble spacer monomers such as (meth)acrylamide, alkyl and dialkyl (meth) acrylamides, alkyl (meth)acrylate, vinyl caprolactone and vinyl pyrrolidine. The alkyl and dialkyl substituted monomers preferably have C₁-C₇ alkyl groups, more preferably C₁-C₃ alkyl groups. Other suitable spacers include vinyl esters, vinyl alcohol, maleic anhydride, propylene glycol and ethylene glycol.

Preferably, the cationic polymer is a cationic polysaccharide polymer such as cationic guar, cationic starch, and cationic cellulose. Suitably, such cationic polysaccharide polymers have a molecular weight of from 100,000 g/mol to 2,300,000 g/mol, more preferably from 150,000 g/mol to 2,000,000 g/mol. Such cationic polysaccharide polymers preferably have a cationic charge density from 0.1 to 4 meq/g.

Cationic polysaccharide polymers suitable for use in compositions of this invention include those represented by the general formula:

A-O-[R¹-N⁺(R²)(R³)(R⁴)X⁻]

wherein: A is an anhydroglucose residual group, such as a starch or cellulose anhydroglucose residual. R¹ is an alkylene, oxyalkylene, polyoxyalkylene, or hydroxyalkylene group, or combination thereof. R², R³ and R⁴ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R², R³ and R⁴) is preferably about 20 or less, and X is an anionic counterion.

Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

Other suitable cationic polysaccharide polymers include quaternary nitrogen-containing cellulose ethers (e.g. as described in U.S. Patent 3,962,418) and copolymers of etherified cellulose and starch (e.g. as described in U.S. Patent 3,958,581).

When used, the cationic polymer will generally be present in the hair care composition of the present invention in an amount of from 0.001 to 1% by weight of the hair care composition, more preferably from 0.01 to 0.5%, and most preferably from 0.03 to 0.3%, based on total weight of the hair care composition and including all ranges subsumed therein.

The hair care composition may additionally comprise a conditioning agent to provide conditioning benefit. Preferably, the hair care composition comprises discrete dispersed droplets of a water-insoluble conditioning agent, which has a mean droplet diameter (D_{3,2}) of less than 15 microns, preferably less than 10 microns, more preferably less than 5 microns, most preferably less than 3 microns. The mean droplet diameter (D_{3,2}) of a water-insoluble conditioning agent may be measured by means of a laser light scattering technique, for example using a 2600D Particle Sizer from Malvern Instruments.

The water-insoluble conditioning agent may include non-silicone conditioning agent comprising non-silicone oily or fatty materials such as hydrocarbon oils, fatty esters and mixtures thereof. Preferably, the water-insoluble conditioning agent is emulsified silicone oil.

Suitable silicones include polydiorganosiloxanes, in particular polydimethylsiloxanes which have the CTFA designation dimethicone. Also suitable for use in compositions of this invention (particularly shampoos and conditioners) are polydimethyl siloxanes having hydroxyl end groups, which have the CTFA designation dimethiconol. Also suitable for use in compositions of this invention are silicone gums having a slight degree of crosslinking, as are described for example in WO 96/31188. Preferably, the silicone oil comprises dimethicone, dimethiconol or a mixture thereof.

Suitable emulsified silicones for use in the hair care compositions of this invention are available as pre-formed silicone emulsions from suppliers of silicones such as Dow Corning and GE silicones. The use of such pre-formed silicone emulsion is preferred for ease of processing and control of silicone particle size. Such pre-formed silicone emulsions will typically additionally comprise a suitable emulsifier, and may be prepared by a chemical emulsification process such as emulsion polymerisation, or by mechanical emulsification using a high shear mixer. Examples of suitable pre-formed silicone emulsions include DC1785, DC1788, DC7128, all available from Dow Corning. These are emulsions of dimethiconol/dimethicone.

Another class of silicones which may be used are functionalized silicones such as amino functional silicones, meaning a silicone containing at least one primary, secondary or tertiary amine group, or a quaternary ammonium group. Examples of suitable amino functional silicones include polysiloxanes having the CTFA designation "amodimethicone."

The water-insoluble conditioning agent is generally present in hair care composition of this invention in an amount from 0.05 to 15%, preferably from 0.1 to 10%, more preferably from 0.5 to 8%, most preferably from 1 to 5%, based on total weight of the hair care composition and including all ranges subsumed therein.

Preferably the composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

The suspending agent is generally present in hair care composition of this invention in an amount of from 0.1 to 10%, more preferably from 0.2 to 6%, and most preferably from 0.3 to 4%, based on total weight of the hair care composition and including all ranges subsumed therein.

Preservatives may also be incorporated into the hair care composition of this invention to protect against the growth of potentially harmful microorganisms. Suitable traditional preservatives include alkyl esters of parahydroxybenzoic acid, hydantoin derivatives, propionate salts, and a variety of quaternary ammonium compounds. Illustrative yet non-limiting examples of the types of preservatives that may be used in this invention include, for examples, phenoxyethanol, sodium salicylate, methyl paraben, butyl paraben, propyl paraben, diazolidinyl urea, sodium dehydroacetate, benzyl alcohol, sodium benzoate, iodopropynyl butylcarbamate, caprylyl glycol, disodium EDTA or mixtures thereof. In an especially preferred embodiment, the preservative is sodium benzoate, phenoxyethanol, sodium salicylate or a mixture thereof. Preservatives are preferably employed in amounts ranging from 0.01 to 2% by weight of the hair care composition.

The hair care composition of the present invention may contain other ingredients which are common in the art to enhance physical properties and performances. Suitable ingredients include but are not limited to fragrance, dyes and pigments, pH adjusting agents, pearlescers or opacifiers, viscosity modifiers, thickeners, and natural hair nutrients such as botanicals, fruit extracts, sugar derivatives and amino acids.

### Method and Use

The present invention provides for a non-therapeutic method of treating the scalp or hair comprising application of a composition of the first aspect onto scalp or hair. It is for cosmetic purpose.

The present invention also provides for a method of depositing anti-dandruff agents onto scalp comprising the step of applying the hair care composition according to any of the preceding claims onto scalp surfaces of an individual followed by rinsing the surfaces with water. The anti-dandruff agent is selected from a piroctone compound, climbazole, ketoconazole and mixtures thereof. It is preferred the anti-dandruff agent is a piroctone compound which is piroctone olamine. The method is non-therapeutic in nature. Preferably it is for cosmetic purpose.

The present invention also provides a composition of the first aspect for use in preventing or alleviating the symptoms of dandruff on the scalp or hair.

The present invention also provides a composition of the first aspect for use in a method to mitigate dandruff.

### Mode of Use

The compositions of the invention are primarily intended for topical application to scalp and/or at least a portion of the hair of an individual, either in rinse-off or leave-on compositions, preferably in rinse-off compositions like shampoos.

When the composition is a shampoo, it is topically applied to the hair and then massaged into the hair and scalp. Then it is rinsed with water prior to drying the hair.

The invention will be further illustrated by the following, non-limiting Examples, in which all percentages quoted are by weight based on total weight unless otherwise stated.

The examples are intended to illustrate the invention and are not intended to limit the invention to those examples *per se.*

### Examples

### Example 1

This example demonstrated the combination of coconut oil and caprylhydroxamic acid can affect the deposition of piroctone olamine onto scalp. The compositions according to the formulations detailed in Table 1 are prepared. All ingredients are expressed by weight percent of the total formulation, and as level of active ingredient.

**TABLE 1**

| **Ingredient** | **Samples** | | | | | | | | |
|---|---|---|---|---|---|---|---|---|---|
| | **A** | **B** | **C** | **D** | **1** | **2** | **3** | **4** | **5** |
| Water | To 100 | To 100 | To 100 | To 100 | To 100 | To 100 | To 100 | To 100 | To 100 |
| Sodium lauryl sulphate | 13 | 13 | 13 | 13 | 13 | 13 | 13 | 13 | 13 |
| Piroctone olamine (Octopirox^{®}) | 0.30 | 0.30 | 0.30 | 0.30 | 0.30 | 0.30 | 0.30 | 0.30 | 0.30 |
| Caprylhydroxamic acid | 0 | 0 | 0.30 | 0.15 | 0.15 | 0.15 | 0.15 | 0.30 | 0.30 |
| Coconut oil | 0 | 0.3 | 0 | 0.09 | 0.15 | 0.30 | 0.45 | 0.30 | 0.60 |
| Carbomer | 0.35 | 0.35 | 0.35 | 0.35 | 0.35 | 0.35 | 0.35 | 0.35 | 0.35 |
| Guar hydroxypropyltrimonium chloride | 0.20 | 0.20 | 0.20 | 0.20 | 0.20 | 0.20 | 0.20 | 0.20 | 0.20 |
| Cocoamidopropyl betaine | 1.50 | 1.50 | 1.50 | 1.50 | 1.50 | 1.50 | 1.50 | 1.50 | 1.50 |
| Perfume | 0.60 | 0.60 | 0.60 | 0.60 | 0.60 | 0.60 | 0.60 | 0.60 | 0.60 |
| Dimethicone and Dimethiconol | 2.00 | 2.00 | 2.00 | 2.00 | 2.00 | 2.00 | 2.00 | 2.00 | 2.00 |
| Preservative | 0.50 | 0.50 | 0.50 | 0.50 | 0.50 | 0.50 | 0.50 | 0.50 | 0.50 |
| Sodium Salicylate | 0.30 | 0.30 | 0.30 | 0.30 | 0.30 | 0.30 | 0.30 | 0.30 | 0.30 |
| Sodium chloride | 0.60 | 0.60 | 0.60 | 0.60 | 0.60 | 0.60 | 0.60 | 0.60 | 0.60 |
| Weight ratio of (coconut oil + CHA) to piroctone olamine | / | / | / | 0.8:1 | 1:1 | 1.5:1 | 2:1 | 2:1 | 3:1 |

### Methods

About 0.2 grams of the test sample was taken on artificial skin (VITRO-SKIN from IMS testing group). This was diluted with 1.8 mL water and rubbed with a plastic rod for 30 seconds. The artificial skin surface was then rinsed twice with water, first time with 4 mL water for 30 second and then again with 4 mL water for 30 seconds. The deposition of piroctone olamine on the skin (10.75 cm² per plate) was measured using HPLC method.

### Results

The average deposition (of five such experiments) is summarized in Table 2.

**TABLE 2**

| **Samples** | **A** | **B** | **C** | **D** | **1** | **2** | **3** | **4** | **5** |
|---|---|---|---|---|---|---|---|---|---|
| Piroctone olamine deposition (µg/plate) | 4.02 | 3.57 | 4.06 | 3.92 | 4.26 | 5.63 | 5.91 | 5.40 | 4.15 |

Samples 1-5 within the scope of the invention showed better deposition of piroctone olamine as compared to samples A to D (out of the scope of the invention).

### Example 2

This example demonstrated the combination of sunflower oil and caprylhydroxamic acid can affect the deposition of piroctone olamine onto scalp. The compositions according to the formulations detailed in Table 3 are prepared. All ingredients are expressed by weight percent of the total formulation, and as level of active ingredient.

**TABLE 3**

| **Ingredient** | **Samples** | | | | |
|---|---|---|---|---|---|
| | **E** | **F** | **6** | **7** | **8** |
| Water | To 100 | To 100 | To 100 | To 100 | To 100 |
| Sodium lauryl sulphate | 13 | 13 | 13 | 13 | 13 |
| Piroctone olamine (Octopirox^{®}) | 0.30 | 0.30 | 0.30 | 0.30 | 0.30 |
| Caprylhydroxamic acid | 0 | 0.45 | 0.15 | 0.30 | 0.45 |
| Sunflower oil | 0 | 2.1 | 0.15 | 0.15 | 0.9 |
| Carbomer | 0.35 | 0.35 | 0.35 | 0.35 | 0.35 |
| Guar hydroxypropyltrimonium chloride | 0.20 | 0.20 | 0.20 | 0.20 | 0.20 |
| Cocoamidopropyl betaine | 1.50 | 1.50 | 1.50 | 1.50 | 1.50 |
| Perfume | 0.6 | 0.6 | 0.6 | 0.6 | 0.6 |
| Dimethicone and Dimethiconol | 2.00 | 2.00 | 2.00 | 2.00 | 2.00 |
| Preservative | 0.50 | 0.50 | 0.50 | 0.50 | 0.50 |
| Sodium Salicylate | 0.30 | 0.30 | 0.30 | 0.30 | 0.30 |
| Sodium chloride | 0.60 | 0.60 | 0.60 | 0.60 | 0.60 |
| Weight ratio of (Sunflower oil + CHA) to piroctone olamine | / | 8.5:1 | 1:1 | 1.5:1 | 4.5:1 |

### Methods

Use the same method as introduced in Example 1.

### Results

The average deposition (of five such experiments) is summarized in Table 4.

**TABLE 4**

| **Samples** | **E** | **F** | **6** | **7** | **8** |
|---|---|---|---|---|---|
| Piroctone olamine deposition (µg/plate) | 5.54 | 4.38 | 7.13 | 7.15 | 6.43 |

Samples 6-8 within the scope of the invention showed better deposition of piroctone olamine as compared to samples E-F (out of the scope of the invention).

### Example 3

The example demonstrates the effect of caprylhydroxamic acid in the combination on the deposition of piroctone olamine onto scalp comparing with other aliphatic acid. The compositions according to the formulations detailed in Table 5 are prepared. All ingredients are expressed by weight percent of the total formulation, and as level of active ingredient.

**TABLE 5**

| **Ingredient** | **Samples** | |
|---|---|---|
| | **G** | **9** |
| Water | To 100 | To 100 |
| Sodium lauryl sulphate | 8.5 | 8.5 |
| Piroctone olamine (Octopirox^{®}) | 0.5 | 0.5 |
| Caprylhydroxamic acid | | 0.25 |
| Caprylic Acid | 0.25 | |
| Coconut oil | 0.25 | 0.25 |
| Cocoamidopropyl betaine | 2.3 | 2.3 |
| pH | 6 | 6 |
| Weight ratio of (coconut oil + aliphatic acid) to piroctone olamine | 1:1 | 1:1 |
| Weight ratio of aliphatic acid to coconut oil | 1:1 | 1:1 |

### Methods

Use the same deposition evaluation method as introduced in Example 1.

### Results

The average deposition (of five such experiments) is summarized in Table 6.

**TABLE 6**

| **Samples** | **G** | **9** |
|---|---|---|
| Piroctone olamine deposition (µg/plate) | 14.79 | 19.15 |

Sample 9 within the scope of the invention showed better deposition of piroctone olamine as compared to sample G (out of the scope of the invention).

## Claims

1. A hair care composition comprising:
i) a cleansing surfactant comprising an anionic surfactant and an amphoteric surfactant;
ii) a hydroxamic acid or salt thereof having a carbon chain of C4 to C12 in an amount of from 0.01 to 10% by weight of the total composition;
iii) an oil phase comprising at least one liquid hydrocarbon oil in an amount of from 0.01 to 10% by weight of the total composition; and
iv) a piroctone compound;
wherein the weight ratio of the amount of the combination of said hydroxamic acid or salt and said liquid hydrocarbon oil to the amount of said piroctone compound is from 1:1 to 8:1; wherein said hydroxamic acid is caprylhydroxamic acid.

2. A composition as claimed in claim 1 wherein said liquid hydrocarbon oil is selected from the group consisting of triglycerides and fatty esters, preferably selected from the group consisting of triheptanoin, tricaprylin, tricaprin, triundecanoin, trilinolein, triolein, almond oil, coconut oil, olive oil, palm kernel oil, peanut oil and sunflower oil, more preferably selected from coconut oil and sunflower oil.

3. A composition as claimed in claim 1 or 2 wherein said liquid hydrocarbon oil is coconut oil or sunflower oil.

4. A composition as claimed in any of claims 1 to 3 wherein said anionic surfactant is an ethoxylated alkyl sulphate a degree of ethoxylation of less than 3, preferably an ethoxylated alkyl sulphate a degree of ethoxylation of less than 2, more preferably sodium laureth sulphate (1EO).

5. A composition as claimed in any of claims 1 to 4 wherein said piroctone compound is piroctone olamine.

6. A composition as claimed in any of claims 1 to 5 wherein the composition comprises piroctone compound in an amount of from 0.01 to 10% by weight of the total composition, preferably from 0.1 to 5%, more preferably from 0.2 to 3%.

7. A composition as claimed in any of claims 1 to 6 wherein the composition comprises said liquid hydrocarbon oil in an amount of from 0.1 to 5%, preferably from 0.1 to 2%, by weight of the total composition.

8. A composition as claimed in any of claims 1 to 7 wherein the composition comprises said hydroxamic acid or salt thereof in an amount of from 0.1 to 5%, preferably from 0.1 to 2%, by weight of the total composition.

9. A composition as claimed in any of claims 1 to 8 wherein the composition comprises a total cleansing surfactant level from 3 to 35 wt%, preferably from 5 to 25 wt%, most preferably from 7 to 16 wt% of the total composition.

10. A composition as claimed in any of claims 1 to 9 wherein the composition comprises a cationic polymer.

11. A composition as claimed in any of claims 1 to 10 wherein the composition is a shampoo.

12. A non-therapeutic method of treating the scalp or hair comprising application of a composition as claimed in any of claims 1 to 11 to the scalp or hair.

13. A non-therapeutic method of depositing piroctone compound onto scalp comprising the step of applying the hair care composition as claimed in any of claims 1 to 11 onto scalp surfaces of an individual followed by rinsing the surfaces with water.

14. A composition as claimed in any of claims 1 to 11 for use in preventing or alleviating the symptoms of dandruff on the scalp or hair.

## Patentansprüche

1. Haarpflegezusammensetzung, umfassend:
i) ein Reinigungstensid, das ein anionisches Tensid und ein amphoteres Tensid umfasst;
ii) eine Hydroxamsäure oder ein Salz davon mit einer Kohlenstoffkette von C4 bis C12 in einer Menge von 0,01 bis 10 Gewichts-% der Gesamtzusammensetzung;
iii) eine Ölphase, die mindestens ein flüssiges Kohlenwasserstofföl in einer Menge von 0,01 bis 10 Gewichts-% der Gesamtzusammensetzung umfasst; und
iv) eine Piroctonverbindung;
wobei das Gewichtsverhältnis der Menge der Kombination der Hydroxamsäure oder des Salzes und des flüssigen Kohlenwasserstofföls zu der Menge der Piroctonverbindung 1:1 bis 8:1 beträgt, wobei die Hydroxamsäure Caprylhydroxamsäure ist.

2. Zusammensetzung, wie im Anspruch 1 beansprucht, wobei das flüssige Kohlenwasserstofföl aus der Gruppe ausgewählt ist, bestehend aus Triglyceriden und Fettestern, bevorzugt aus der Gruppe ausgewählt ist, bestehend aus Triheptanoin, Tricaprylin, Tricaprin, Triundecanoin, Trilinolein, Triolein, Mandelöl, Kokosnussöl, Olivenöl, Palmkernöl, Erdnussöl und Sonnenblumenöl, bevorzugter ausgewählt aus Kokosnussöl und Sonnenblumenöl.

3. Zusammensetzung, wie in Anspruch 1 oder 2 beansprucht, wobei das flüssige Kohlenwasserstofföl Kokosnussöl oder Sonnenblumenöl ist.

4. Zusammensetzung, wie in einem der Ansprüche 1 bis 3 beansprucht, wobei das anionische Tensid ein ethoxyliertes Alkylsulfat eines Ethoxylierungsgrades von weniger als 3, bevorzugt ein ethoxyliertes Alkylsulfat eines Ethoxylierungsgrades von weniger als 2, bevorzugter Natriumlaurethsulfat (1 EO) ist.

5. Zusammensetzung, wie in einem der Ansprüche 1 bis 4 beansprucht, wobei die Piroctonverbindung Piroctonolamin ist.

6. Zusammensetzung, wie in einem der Ansprüche 1 bis 5 beansprucht, wobei die Zusammensetzung die Piroctonverbindung in einer Menge von 0,01 bis 10 Gewichts-% der Gesamtzusammensetzung, bevorzugt von 0,1 bis 5%, bevorzugter von 0,2 bis 3% umfasst.

7. Zusammensetzung, wie in einem der Ansprüche 1 bis 6 beansprucht, wobei die Zusammensetzung das flüssige Kohlenwasserstofföl in einer Menge von 0,1 bis 5%, bevorzugt von 0,1 bis 2%, bezogen auf das Gewicht der Gesamtzusammensetzung, umfasst.

8. Zusammensetzung, wie in einem der Ansprüche 1 bis 7 beansprucht, wobei die Zusammensetzung die Hydroxamsäure oder das Salz davon in einer Menge von 0,1 bis 5%, bevorzugt von 0,1 bis 2%, bezogen auf das Gewicht der Gesamtzusammensetzung, umfasst.

9. Zusammensetzung, wie in einem der Ansprüche 1 bis 8 beansprucht, wobei die Zusammensetzung einen Gesamtgehalt an Reinigungstensid von 3 bis 35 Gew.-%, bevorzugt von 5 bis 25 Gew.-%, höchst bevorzugt von 7 bis 16 Gew.-% der Gesamtzusammensetzung umfasst.

10. Zusammensetzung, wie in einem der Ansprüche 1 bis 9 beansprucht, wobei die Zusammensetzung ein kationisches Polymer umfasst.

11. Zusammensetzung, wie in einem der Ansprüche 1 bis 10 beansprucht, wobei die Zusammensetzung ein Shampoo ist.

12. Nicht-therapeutisches Verfahren zur Behandlung der Kopfhaut oder des Haars, das das Auftragen einer Zusammensetzung, wie in einem der Ansprüche 1 bis 11 beansprucht, auf die Kopfhaut oder das Haar umfasst.

13. Nicht-therapeutisches Verfahren zum Auftragen einer Piroctonverbindung auf die Kopfhaut, umfassend den Schritt des Aufbringens der Haarpflegezusammensetzung, wie in einem der Ansprüche 1 bis 11 beansprucht, auf die Kopfhautoberflächen einer Person, gefolgt vom Abspülen der Oberflächen mit Wasser.

14. Zusammensetzung, wie in einem der Ansprüche 1 bis 11 beansprucht, zur Verwendung bei der Vorbeugung oder Linderung der Symptome von Schuppen auf der Kopfhaut oder dem Haar.

## Revendications

1. Composition de soin capillaire comprenant :
i) un tensioactif nettoyant comprenant un tensioactif anionique et un tensioactif amphotère ;
ii) un acide hydroxamique ou un sel de celui-ci ayant une chaîne carbonée en C4 à C12 en une quantité de 0,01 à 10 % en poids de la composition totale ;
iii) une phase huileuse comprenant au moins une huile hydrocarbonée liquide en une quantité de 0,01 à 10 % en poids de la composition totale ; et
iv) un composé piroctone ;
dans laquelle le rapport en poids de la quantité de la combinaison dudit acide hydroxamique ou sel et de ladite huile hydrocarbonée liquide à la quantité dudit composé piroctone est de 1/1 à 8/1, dans laquelle ledit acide hydroxamique est l'acide caprylhydroxamique.

2. Composition selon la revendication 1, dans laquelle ladite huile hydrocarbonée liquide est choisie dans le groupe constitué par les triglycérides et les esters gras, de préférence choisie dans le groupe constitué par la triheptanoïne, la tricapryline, la tricaprine, la triundécanoïne, la trilinoléine, la trioléine, l'huile d'amande, l'huile de coco, l'huile d'olive, l'huile de palmiste, l'huile d'arachide et l'huile de tournesol, mieux encore choisie parmi l'huile de coco et l'huile de tournesol.

3. Composition selon la revendication 1 ou 2, dans laquelle ladite huile hydrocarbonée liquide est l'huile de coco ou l'huile de tournesol.

4. Composition selon l'une quelconque des revendications 1 à 3, dans laquelle ledit tensioactif anionique est un alkylsulfate éthoxylé ayant un degré d'éthoxylation inférieur à 3, de préférence un alkylsulfate éthoxylé ayant un degré d'éthoxylation inférieur à 2, mieux encore le laureth-sulfate de sodium (1EO).

5. Composition selon l'une quelconque des revendications 1 à 4, dans laquelle ledit composé piroctone est la piroctone olamine.

6. Composition selon l'une quelconque des revendications 1 à 5, laquelle composition comprend le composé piroctone en une quantité de 0,01 à 10 %, de préférence de 0,1 à 5 %, mieux encore de 0,2 à 3 %, en poids de la composition totale.

7. Composition selon l'une quelconque des revendications 1 à 6, laquelle composition comprend ladite huile hydrocarbonée liquide en une quantité de 0,1 à 5 %, de préférence de 0,1 à 2 %, en poids de la composition totale.

8. Composition selon l'une quelconque des revendications 1 à 7, laquelle composition comprend ledit acide hydroxamique ou sel de celui-ci en une quantité de 0,1 à 5 %, de préférence de 0,1 à 2 %, en poids de la composition totale.

9. Composition selon l'une quelconque des revendications 1 à 8, laquelle composition comprend un taux de tensioactif nettoyant total de 3 à 35 % en poids, de préférence de 5 à 25 % en poids, tout spécialement de 7 à 16 % en poids de la composition totale.

10. Composition selon l'une quelconque des revendications 1 à 9, laquelle composition comprend un polymère cationique.

11. Composition selon l'une quelconque des revendications 1 à 10, laquelle composition est un shampooing.

12. Procédé non thérapeutique de traitement du cuir chevelu ou des cheveux, comprenant l'application d'une composition selon l'une quelconque des revendications 1 à 11 sur le cuir chevelu ou les cheveux.

13. Procédé non thérapeutique de déposition d'un composé piroctone sur le cuir chevelu, comprenant l'étape d'application de la composition de soin capillaire selon l'une quelconque des revendications 1 à 11 sur les surfaces du cuir chevelu d'un individu, suivie d'un rinçage à l'eau des surfaces.

14. Composition selon l'une quelconque des revendications 1 à 11, pour une utilisation dans la prévention ou l'atténuation des symptômes de pellicules sur le cuir chevelu ou les cheveux.
